# EUROPEAN PATENT APPLICATION

(11) **EP 0 736 304 A1**
(43) Date of publication of application: **09.10.1996**
(21) Application number: 96830122.6
(22) Date of filing: 18.03.1996
(51) Int. Cl.: A61M 5/14

(54) **Drip chamber assembly for infusion/transfusion lines**

(30) Priority: 06.04.1995 IT TO950088 U
(71) Applicant: INDUSTRIE BORLA S.p.A., I-10024 Moncalieri (Torino) (IT)
(72) Inventor: Guala, Ernesto, 10133 Torino (IT); Guala, Gianni, 10133 Torino (IT)
(74) Representative: Buzzi, Franco

(57) **Abstract**

A drip chamber assembly (1) for infusion/transfusion lines, to be used with a control and automatic metering apparatus, comprising a first and a second hollow elements (2,3) having respective open ends (8,13) for their mutual axial hermetic connection at which an outer annular collar is provided, which is intended to be coupled through a restrained connection to a body (C) carrying an optical-electrical detector of the control and metering apparatus. The annular collar is formed as an integral projection (9) of the first hollow element (2), in correspondence of which the connecting end (13) of the second hollow element (3) is fixed by glueing (16) to the connection end (8) of the first hollow element (2). The integral projection (9) can be further employed for the attachment of a suspension hanger (17) between the perforating-dripping assembly (1) and a container of the infusion/transfusion liquid (B)

## Description

The present invention is related to a perforating-dripping assembly for infusion/transfusion medical lines, intended to be used for the metered supply of an infusion/transfusion liquid contained within a suspended container.

More particularly, the invention is directed to a perforating-dripping assembly of the type comprising a first hollow element and a second hollow element provided with respective open ends for their mutual axial hermetic connection, the first hollow element being formed at its opposite end with a perforating tip and the second hollow element being intended to be connected at its opposite end to said infusion/transfusion line.

For the metered supply of the infusion/transfusion liquid from the related container to the user, the liquid flow-line is traditionally provided with manual adjustment systems of the liquid which, through the perforating tip, drips into the chamber defined by the two mutually connected hollow elements of the assembly.

More recently, to the aim of carrying out automatically and very precisely the metered supply of the infusion/transfusion liquid, control and metering apparatus have been made available on the market, which include a positive-displacement pump operatively associated to the liquid flow line, and an electronic control system electrically connected to a body carrying an optical-electrical detector of the liquid dripping trough the perforating tip of the assembly. This body is formed with an engagement seat for anchoring thereof astride of the perforating-gripping assembly.

In this case the perforating-dripping assembly is to be provided with an outer annular collar, conveniently arranged in correspondence of said mutual connection ends of the first and second hollow elements, to be engaged through a restrained connection within said seat of the body carrying the optical-electrical detector of the control and automatic metering apparatus.

In a known perforating-dripping assembly, which is presently available on the market, the outer annular collar is constituted by a distinct and separate member, which is applied by over-moulding in correspondence of respective outer radial flanges formed at the mutual connection ends of the first and second hollow elements of the assembly, and by means of which these mutual connection ends are hermetically secured to each other.

This arrangement is relatively complicated and expensive, since it involves, for the manufacturing of the perforating-dripping assembly, an additional over-moulding step of the above-mentioned collar.

A further drawback of this known arrangement resides in that, in order to ensure in use a reliable connection between the perforating-dripping assembly and the suspended container of the infusion/transfusion liquid, it is expedient to provide a mutual anchoring system so as to prevent any risks of disengagement thereof and consequent fall of the perforating-dripping assembly relative to the suspended container. Actually, the chances of disengagement are relatively high owing to the concomitance of two circumstances: the first one is related to the fact that, in order to enhance penetration of the perforating tip across a pierceable cap of the infusion/transfusion liquid container, the perforating tip is normally treated superficially with low-friction-coefficient substances, for instance a silicone coating, which may however promote withdrawal under gravity of the assembly from the container cap in the suspended condition of use. The second one is related to the fact that the body with the optical-electrical detector of the control and metering apparatus, which is arranged in use astride of the perforating-dripping assembly, is relatively heavy and is thus contributing to gravity withdrawal of the perforating tip from the container of the infusion/transfusion liquid.

According to the above known construction, which as previously explained employs an over-moulded ring as the collar for engagement of the perforating-dripping assembly within the seat of the body carrying the optical-electrical detector, provision of a safety system for an auxiliary connection between the assembly and the liquid container is difficult, and requires anyway relatively complicated expedients.

The object of the present invention is to overcome the above drawbacks, and to provide a perforating-dripping assembly of the type set forth at the beginning, which is of more convenient and functional construction and use.

According to its primary feature, the perforating-dripping assembly of the invention is characterised in that said annular collar is formed by an integral projection of said first hollow element, radially projecting outwardly and to which said connection end of the second hollow element is fixed by glueing.

By virtue of this solution, the provision of a perforating-dripping assembly which is suitable for use in connection with a control and automatic metering apparatus of the infusion/transfusion liquid requires no over-moulding operations to form a distinct joining collar between the two hollow elements of the assembly, whose manufacturing is thus more simple and cheap.

Additionally, said integral projection can be easily formed so as to enable in use convenient and easy suspended connection between the perforating-dripping assembly and the liquid container. To such effect, according to a preferred embodiment of the invention, said integral projection is hollow and is formed with a stepped wall defining two annular cavities which are open towards the second hollow element and are axially offset relative to each other, of which the radially inner annular cavity houses said connection end of the second hollow element, and the radially outer cavity defines an anchoring seat for an auxiliary safety connection means of the perforating-dripping assembly to the container of the infusion /transfusion liquid.

For such an auxiliary connection, the perforating-dripping assembly may conveniently further comprise a suspension hanger having hooked ends to be engaged one into said anchoring seat of said integral projection, and the other to an attachment member of the container of the infusion-transfusion liquid.

The invention will now be disclosed in detail with reference to the accompanying drawings, purely provided by way of non-limiting example, in which:
- Figure 1 is a diagrammatic perspective view of a perforating-dripping assembly according to the invention, shown in use, and

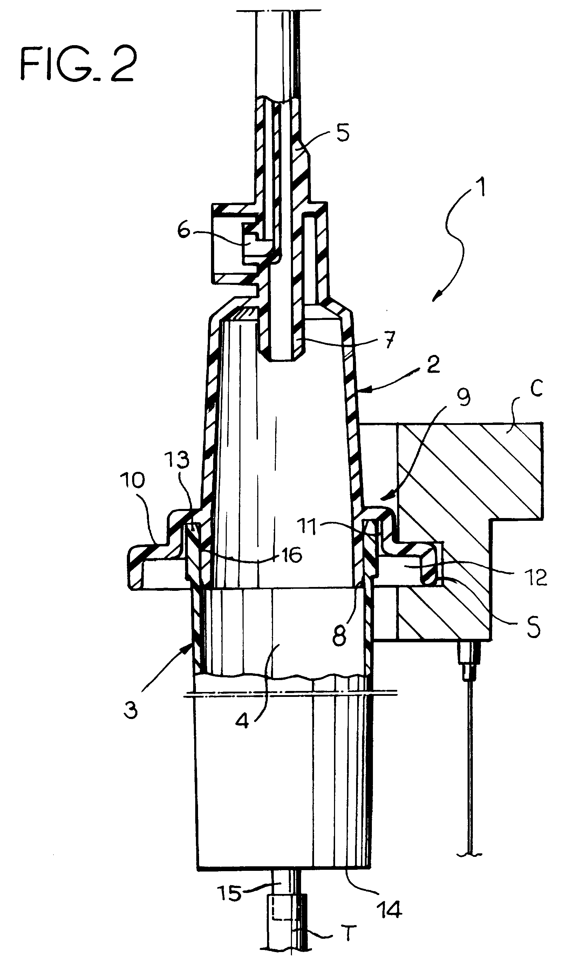

- Figure 2 is a partially vertically sectioned and enlarged view along line II-II of figure 1.

In the drawings, reference numeral 1 generally designates a perforating-dripping assembly according to the invention, for infusion/transfusion medical lines. The assembly 1 is constituted by a first hollow element 2 and a second hollow element 3, both made of transparent moulded plastic material and hermetically joined to each other, in the way clarified herebelow, so as to define a dripping chamber 4.

The first hollow element 2 is integrally formed at one end with a tubular perforating tip 5, having a lateral vent port 6 in correspondence of which a filtering member with associated closing cap, not shown in the drawings since conventional, is normally arranged. The perforating tip 5 is communicating with the dripping chamber 4 through an inner axial tubular appendage 7.

On the opposite side, the first hollow element 2 has an open end 8 and, at a short distance therefrom, an integrally formed outer projection 9 defining an annular collar the function of which will be clarified in the following. The integral projection 9 is radially protruding outwardly and is made hollow, with a stepped wall 10 defining, together with the outer wall of the corresponding axial portion of the open end 8, a radially inner annular cavity 11 and a radially outer annular cavity 12, axially offset from each other and open towards the second hollow element 3.

The second hollow element 3 has one open and thickened end 13, the inner diameter of which is substantially corresponding to the outer diameter of the open end 8 of the first hollow element 2, and is closed at the opposite end by a bottom wall 14 from which an outer tubular connector 15 is axially projecting.

The open end 13 of the second hollow element 3 is axially fitted over the open end 8 of the first hollow element 2, within the radially inner annular cavity 11 of the integral projection 9. The two hollow elements 2 and 3 are permanently and hermetically secured to each other by glueing in correspondence of an annular area 16 comprised between the outer surface of the open end 8 and the inner surface of the open end 13.

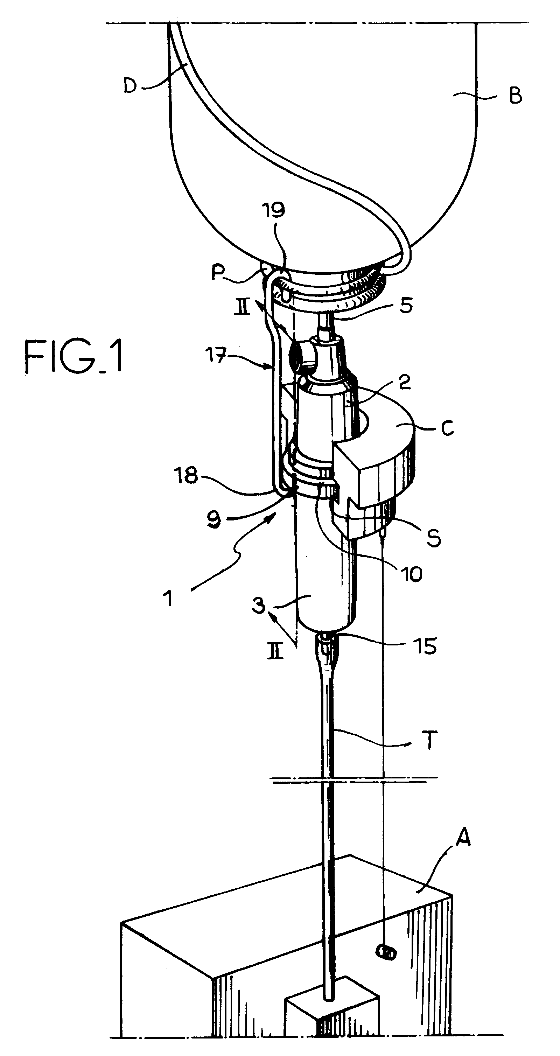

The above-disclosed construction of the integral hollow projection 9 enables conveniently employing the perforating-dripping assembly 1 with a control and automatic metering apparatus of the infusion/transfusion liquid within a container B. This apparatus, generally and partially shown as A in figure 1, is of a conventional type and, therefore, will not be disclosed in detail for the sake of brevity. In connection with the present invention, it is sufficient to point out that the apparatus A (for instance manufactured and marketed by B BRAUN under the trade name INFUSOMAT SECURA ®, or by IMED under the trade name GEMINI PC-1 ®) comprises a positive-displacement metering pump and an electronic control system operatively connected to a half-ring body C incorporating an optical-electrical detector, normally a photoelectric cell transducer, to monitor dripping of the liquid into the chamber 4 of the perforating-dripping assembly 1. As best shown in figure 1, the half-ring body C has an inner groove S defining an engagement seat for anchoring thereof astride of the perforating-dripping assembly 1.

In the condition of use depicted in the drawings, the perforating tip 5 is introduced across a pierceable closure cap, not shown in the drawings since conventional, of the container B, and the tubular connector 5 is connected to a flexible hose or line T. The container B is thus suspended to a support, with the perforating-dripping assembly 1 in turn suspended to the container B, and the hose T is passed within the control and automatic metering apparatus A.

The body C with the optical-electrical detector is then anchored astride of the assembly 1, simply engaging the wall of the integral projection 9, along half of the stepped wall 10 thereof, within the half-annular seat S. The axial size of the seat S and of the corresponding engaging portion of the integral projection 9 will be such to provide a slightly forced mutual engagement therebetween.

Through the transparent material of the perforating-dripping assembly 1, the optical-electrical detector of the body C, which is electrically connected to the apparatus A, performs as explained monitoring of dripping of the liquid from the container C into the chamber 4.

To the aim of preventing in use any risks of accidental disengagement and separation under gravity of the perforating-dripping assembly 1 together with the body C relative to the liquid container B, the invention provides an auxiliary device for mechanical connection between the integral projection 9 on one side, and an attachment member P on the other side, which is for instance part of a retaining and suspension device D, known per se, of the container B.

This attachment member is constituted, in the shown example, by a suspension hanger 17 having opposite hooked ends of which the lower one, designated as 18, is engaged within a corresponding part of the radially outer cavity 12 of the integral projection 9, and the upper one, shown as 19, is anchored to the attachment element P of the container B.

It is to be pointed out that the above-disclosed attachment member may have a different constructive arrangement, anyway adapted to be anchored to the perforating-dripping assembly 1 below the integral projection 9.

Naturally, the details of construction and the embodiments may be widely varied with respect to what has been disclosed and illustrated, without thereby departing from the scope of the present invention, such as defined in the appended claims.

## Claims

1. A perforating-dripping assembly (1) for infusion/transfusion medical lines (T) intended to be used for the metered supply of an infusion/transfusion liquid, contained within a suspended container (B), through a control and automatic metering apparatus (A) including a body (C) carrying an optical-electrical detector and formed with an engagement seat (S) for anchoring thereof astride of the perforating-dripping assembly (1), said perforating-dripping assembly comprising a first hollow element (2) and a second hollow element (3) provided with respective open ends (8,13) of mutual axial hermetic connection, the first hollow element (2) being formed at its opposite end with a perforating tip (5) and the second hollow element (3) being adapted to be connected at its opposite end to said infusion/transfusion line (7), and an outer annular collar (9) arranged in correspondence of said mutual connection ends (8,13) of said first and said second hollow elements (2,3) and engageable through a restrained connection into said engagement seat (S) of said body (C) carrying the optical-electrical detector, characterised in that said annular collar is formed as an integral projection (9) of said first hollow element (2), radially protruding outwardly and to which said connection end (13) of the second hollow element (13) is fixed by glueing (16).

2. Perforating-dripping assembly according to claim 1, characterised in that said integral projection (9) is hollow and has a stepped wall (10) defining two annular cavities (11,12) which are open towards said second hollow element (3) and are axially offset relative to each other, the radially inner annular cavity (11) housing said connection end (13) of the second hollow element (3), and the radially outer cavity (12) defining an anchoring seat for auxiliary safety connecting means of the perforating-dripping assembly to the container (B) of the infusion/transfusion liquid.

3. Perforating-dripping assembly according to claim 2, characterised in that it further comprises a suspension hanger (17) having hooked ends (18,19) to be engaged one into said anchoring seat (12) of said integral projection (9) and the other to an attachment member (P) of the container (B) of the infusion/transfusion liquid.
